(19) Europäisches Patentamt
European Patent Office
Office européen des brevets

(11) Veröffentlichungsnummer: **0 033 459**
**A1**

(12) # EUROPÄISCHE PATENTANMELDUNG

(21) Anmeldenummer: 81100328.4

(22) Anmeldetag: 17.01.81

(51) Int. Cl.³: C 07 D 263/56
D 06 L 3/12

(30) Priorität: 31.01.80 DE 3003540

(43) Veröffentlichungstag der Anmeldung: 12.08.81 Patentblatt 81/32

(84) Benannte Vertragsstaaten: CH DE FR GB IT LI

(71) Anmelder: BAYER AG
Zentralbereich Patente, Marken und Lizenzen
D-5090 Leverkusen 1, Bayerwerk(DE)

(72) Erfinder: Bremen, Josef, Dr.
Am Kettnersbusch 1 e
D-5090 Leverkusen 3(DE)

(72) Erfinder: Dorlars, Alfons, Dr.
Mozartstrasse 32
D-5090 Leverkusen 1(DE)

(72) Erfinder: Schellhammer, Carl-Wolfgang, Dr.
Katharinental 26
D-5060 Bergisch-Gladbach 2(DE)

(72) Erfinder: Wehling, Bernhard, Dr.
Andreas-Gryphius-Strasse 26
D-5000 Köln 80(DE)

(54) **Benzoxazolylstilbene, Verfahren zu deren Herstellung und deren Verwendung als optische Aufheller.**

(57) Verbindungen der Formel

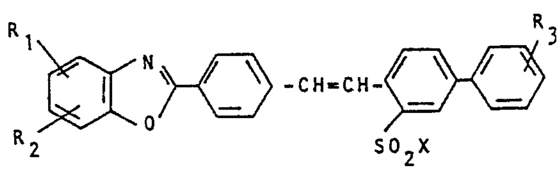

worin
- $R_1$ Wasserstoff, Alkyl, Halogen, Alkoxy, Cycloalkyl, Aralkyl, alkylsulphonyl, Phenylsulfonyl, COOV, $SO_3W$, CONVW oder $SO_2NVW$,
- $R_2$ Wasserstoff, Alkyl, Alkoxy, Halogen
- $R_1$ und $R_2$ gemeinsam einen annelierten carbocyclischen Ring,
- $R_3$ Wasserstoff, Halogen, COOV, $SO_3W$, CONVW oder $SO_2NVW$,
- X NVW, Z oder OV,
- V Wasserstoff oder Z
- W Wasserstoff oder Alkyl und
- Z Alkyl oder Phenyl bedeuten,

sind vielseitig anwendbare optische Aufheller. Hervorzuheben sind Verbindungen dieser Formel mit X = OH, welche sich ausgezeichnet zur Einarbeitung in Waschmittel eignen. Die Herstellung erfolgt nach üblichen Verfahren.

EP 0 033 459 A1

Croydon Printing Company Ltd.

BAYER AKTIENGESELLSCHAFT 5090 Leverkusen-Bayerwerk
Zentralbereich
Patente Marken und Lizenzen K/ABc

# Benzoxazolylstilbene, Verfahren zu deren Herstellung und deren Verwendung als optische Aufheller

Gegenstand der Erfindung sind Verbindungen der Formel

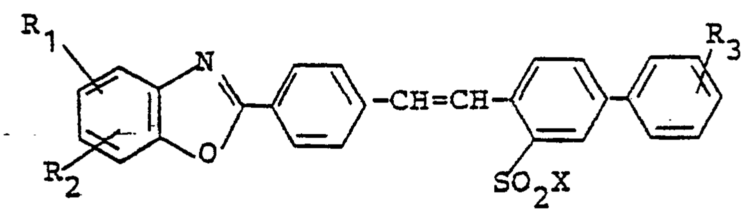

(I)

worin

$R_1$ Wasserstoff, Alkyl, Halogen, Alkoxy, Cycloalkyl, Aralkyl, Alkylsulfonyl , Phenylsulfonyl, COOV, $SO_3W$, CONVW oder $SO_2NVW$,

$R_2$ Wasserstoff, Alkyl, Alkoxy, Halogen

$R_1$ und $R_2$ gemeinsam einen annelierten carbocyclischen Ring,

$R_3$ Wasserstoff, Halogen, COOV, $SO_3W$, CONVW oder $SO_2NVW$,

X NVW, Z oder OV,

V Wasserstoff oder Z

W Wasserstoff oder Alkyl und

Z Alkyl oder Phenyl bedeuten,

wobei die vorstehend genannten Kohlenwasserstoff- und Alkoxyreste sowie die gegebenenfalls anellierten Ringsysteme durch in der Aufhellerchemie übliche Substituenten substituiert sein können.

Geeignete beliebige Alkyl- und Alkoxyreste sind solche mit 1 bis 4 C-Atomen, wie z.B. Methyl, Ethyl, Methoxy und Ethoxy.

Geeignete Phenylreste sind gegebenenfalls durch Alkyl, Alkoxy, Halogen, CN, COOR, $SO_2R$ u.a. substituierte Phenylreste.

Unter "Halogen" wird vor allem Brom und insbesondere Chlor verstanden.

Geeignete annelierte Ringe sind gegebenenfalls durch Halogen oder Alkyl substituierte Cyclohexan-, Benzol- oder Naphthalinringe.

Von besonderer technischer Bedeutung sind Verbindungen der Formel (I), worin X für OH bzw. die davon abgeleiteten Salze steht.

Im Rahmen der neuen Verbindungen der Formel I sind weiterhin solche hervorzuheben, die in Form der freien Säure der Formel

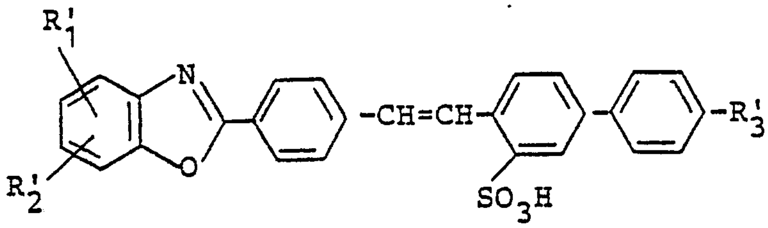

(II)

entsprechen, worin

$R_1'$ Wasserstoff, $C_1$-$C_4$-Alkyl, $C_1$-$C_4$-Alkoxy, Chlor, COOH oder $SO_3H$,

$R_2'$ Wasserstoff, $C_1$-$C_4$-Alkyl oder Chlor und

$R_3'$ Wasserstoff, Chlor, COOH oder $SO_3H$ bedeuten.

"Sperrige" Reste, wie z.B. tert.-Butyl, befinden sich dabei in solchen Positionen, wo sie keine sterische Hinderung bewirken.

Die neuen 4-Benzoxazolyl-4'-phenylstilbene können auf verschiedene Weise hergestellt werden. Ein Verfahren zur Herstellung der Verbindungen der Formel (I) ist dadurch gekennzeichnet, daß man Verbindungen der Formel

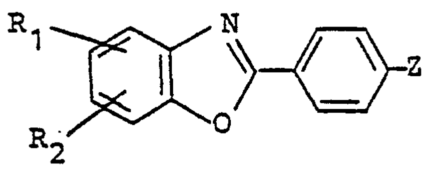

(III)

in der $R_1$ und $R_2$ die angegebene Bedeutung haben und

Z für eine Gruppierung der Formeln

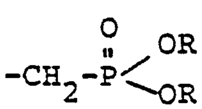 , 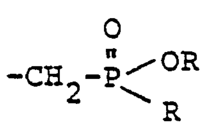 , 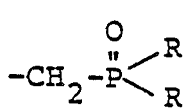 oder 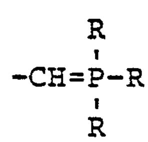

steht, in welchen R ein Alkyl- Aralkyl, Cycloalkyl oder Arylrest ist, mit Verbindungen der Formel

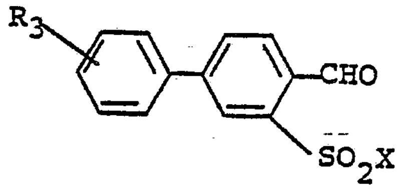

(VI)

in der $R_3$ und X die oben angegebene Bedeutung haben, in Gegenwart einer starken Base in an sich bekannter Weise umsetzt (vgl. DOS 2 525 684).

Die Umsetzung erfolgt zweckmäßigerweise in stark polaren organischen Lösungsmitteln, wie z.B. Dimethylformamid und Dimethylsulfoxid bei Temperaturen von 20 bis 80°C.

Geeignete Basenkatalysatoren sind Alkalihydroxide, -amide, -carbonate und -alkoholate. Auch Phenyllithium und organische Basen, wie Trialkylammoniumhydroxide kommen in Betracht.

Ein anderes Verfahren zur Herstellung der Verbindungen der Formel I ist dadurch gekennzeichnet, daß man nach dem Prinzip der Anil-Synthese eine Schiffsche Base der Formel

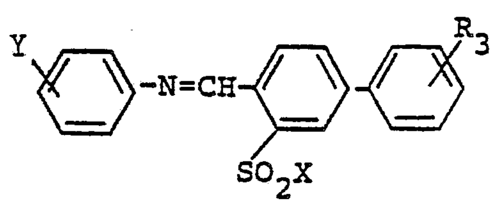

(V)

worin

Y für H oder Cl steht und $R_3$ und X die oben angegebene Bedeutung haben,

mit einer Verbindung der Formel

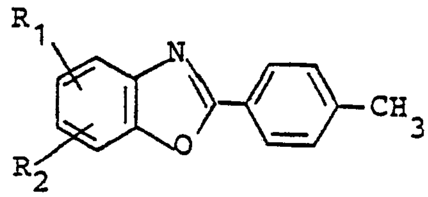

(VI)

in Gegenwart einer starken Base kondensiert.

Einzelheiten zur präparativen Durchführung dieser Verfahrensvariante findet man ebenfalls in der oben zitierten Offenlegungsschrift sowie in DOS 2 525 681 (entsprechen in etwa US-PS 4 014 642, 4 014 644 und 4 014 870).

Außer den bereits oben erwähnten Basen eignen sich für diese Variante vor allem Kaliumalkoholate, wie z.B. Kalium-tertiär-butylat.

Die Umsetzung erfolgt im allgemeinen bei 20-150°C, vorzugsweise 40-80°C.

Bevorzugtes Reaktionsmedium sind die obengenannten polaren Lösungsmittel.

Die Aufarbeitung der Reaktionsgemische erfolgt in üblicher Weise.

Während die als Ausgangsstoffe benötigten Verbindungen der Formeln III und VI allgemein bekannt sind, sind die Aldehyde der Formel IV bzw. deren Schiffsche Basen bislang nicht in der Literatur beschrieben worden.

Man erhält diese Aldehyde aber relativ leicht nach an sich bekannten Verfahren, indem man Verbindungen der Formel

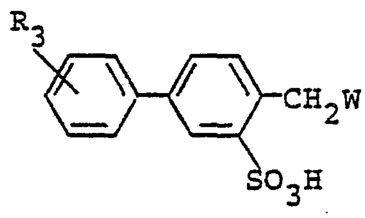

(VII)

worin W für $NH_2$, OH oder Cl steht, oxidiert.

Im Falle von W = $NH_2$ oder Cl erfolgt die Oxidation nach dem Prinzip der Sommelet'schen Aldehyd-Synthese (vgl. Organic Reactions, Vol. 8, p. 197).

Die Hydroxymethylverbindungen der Formel VII werden mit üblichen Oxidationsmitteln, wie Chromsäure, Dichromat oder $MnO_2$, behandelt.

Die Verbindungen der Formel VII erhält man beispielsweise durch katalytische Hydrierung von Verbindungen der Formel

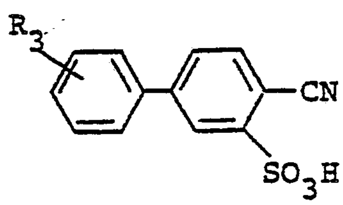

(VIII)

sowie gegebenenfalls anschließende Diazotierung und Verkochung der Aminomethylgruppe und gewünschtenfalls anschließende Halogenierung der resultierenden Hydroxymethylgruppe.

Ein weiteres Verfahren zur Herstellung der Aldehyde der Formel (IV), in der X eine OH-Gruppe und $R_3$ Wasserstoff bedeuten, ist dadurch gekennzeichnet, daß man 2-Chlor-4-phenylbenzaldehyd der Formel

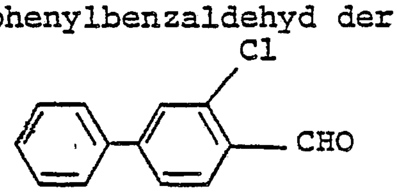

(IX)

mit Natriumsulfit in Wasser bei 150-200°C umsetzt.

Die neuen Benzoxazolylstilbene der Formel I sind gelbstichig gefärbte, in Lösung stark blau fluoreszierende Substanzen, die sich dank eines ausgezeichneten Ziehvermögens vorzüglich zum Aufhellen von organischen Materialien eignen.

Während diejenigen Aufheller-Typen der Formel I, die frei von wasserlöslich machenden Gruppen sind, zum Aufhellen von synthetischen Fasermaterialien (insbesondere solchen aus Polyestern) dienen, werden die bevorzugten, sulfonsäuregruppenhaltigen Verbindungen der Formel I, worin X = OH, zum Aufhellen von Cellulosematerialien eingesetzt.

Besonders geeignet sind die letztgenannten Typen zur Einarbeitung in Waschmittel.

Die Verbindungen der Formel I eignen sich weiterhin als Nuancierungsfarbstoff zur Aspektverbesserung, als Zusatzmittel zu chemischen Bleichmitteln zu sogenannten "master batches", als Szintillatoren oder - je nach Substitution - als Laser-Farbstoffe.

Beispiel 1

6,9 g 2-(4'-Diethoxyphosphonomethylphenyl)-benzoxazol und 9,5 g 4-Phenylbenzaldehyd-2-sulfonsäure, Na-Salz (65 %ig) werden unter Durchleiten von Stickstoff in 100 ml Dimethylformamid auf 40°C erwärmt. In die entstandene Lösung werden bei 40-45°C innerhalb von 45 Min. 11 g einer 30 %igen Natriummethylatlösung in Methanol getropft.

Das Reaktionsgemisch wird noch 4 Stunden bei 45-50°C gerührt. Danach wird auf Raumtemperatur abgekühlt, mit 700 ml Wasser versetzt, zum Sieden erhitzt, abgesaugt und bei 100°C getrocknet. Man erhält 9,1 g (etwa 90 % der Theorie) der Verbindung der Formel (101)

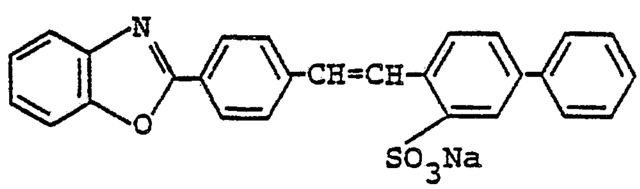

als gelbes Pulver.

Verwendet man anstelle von 2-(4'-Diethoxyphosphonomethylphenyl)-benzoxazol die äquivalente Menge 2-(4'-Diethoxyphosphonomethylphenyl)-5-methyl-benzoxazol der Formel (102)

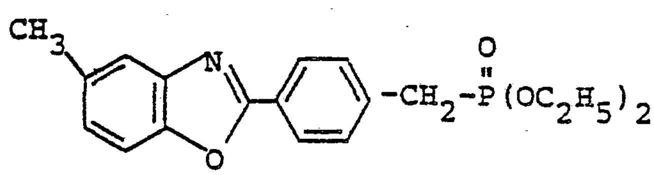

bzw. 2-(4'-Diethoxyphosphonomethylphenyl)-5,7-dimethyl-benzoxazol der Formel (103)

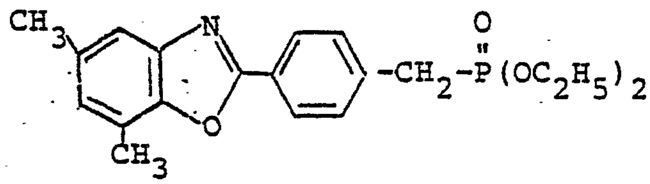

und verfährt im übrigen wie vorstehend beschrieben, so erhält man die Verbindung der Formel (104)

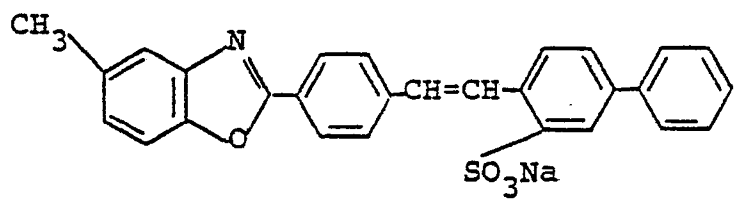

in Form eines gelben Pulvers bzw. die Verbindung der Formel (105)

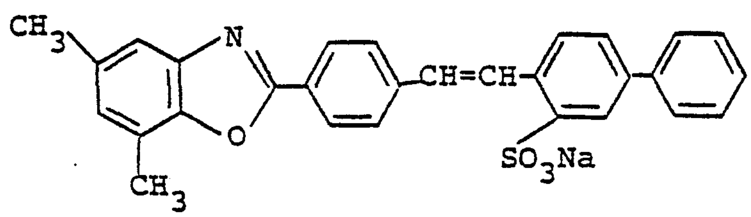

in Form eines gelben Pulvers.

Die Verbindungen lösen sich in Dimethylformamid mit neutral blauer Fluorezzenz am Tageslicht.

Die Herstellung der als Ausgangsprodukte benötigten Phosphonate sei am Beispiel des 2-(4'-Diethoxyphosphonomethylphenyl)-benzoxazols beschrieben:

16 g 2-(4'-Chlormethylphenyl)-benzoxazol (hergestellt durch Kondensation von o-Aminophenol mit 4-Chlormethylbenzoylchlorid und anschließendem Ringschluß in Xylol in Gegenwart von p-Toluolsulfonsäure, Schmelzpunkt 146-148°C) werden mit 100 ml Triethylphosphit innerhalb von 2 Stunden auf 155-160°C erhitzt und anschließend noch 8 Stunden bei 160°C gerührt. Nach dem Abkühlen auf 90°C wird das überschüssige Triethylphosphit im Vakuum abdestilliert und der Rückstand aus Cyclohexan umkristallisiert und getrocknet. Man erhält 19,4 g (86 % d.Th.) 2-(4'-Diethoxyphosphonomethylphenyl)-benzoxazol vom Schmelzpunkt 66 bis 69°C.

Das als Ausgangsprodukt benötigte 4-Phenylbenzaldehyd-2-sulfonsäure, Na-Salz wird wie folgt hergestellt:

100 g 4-Cyano-biphenyl-3-sulfonsäure, Na-Salz werden in 1500 ml 80 %igem Methanol heiß gelöst und dann bei 60°C und 90 bar Wasserstoff-Druck in Gegenwart von 20 g Raney-Nickel und 100 ml flüssigem Ammoniak hydriert. Nach Beendigung der Wasserstoffaufnahme wird auf Raumtemperatur abgekühlt, entspannt, vom Katalysator abfiltriert und das Lösungsmittel abdestilliert. Der feste Rückstand wird in wenig Wasser gelöst, geklärt, durch Ansäuern ausgefällt und getrocknet. Man erhält 61 g 4-Aminomethyl-biphenyl-3-sulfonsäure.

26,3 g der oben erhaltenen 4-Aminomethyl-biphenyl-3-sulfonsäure werden in 200 ml 80 %iger Essigsäure suspendiert. Hierauf werden 15 ml 30 %ige wäßrige Formalinlösung und 16,5 g Urotropin zugegeben. Es wird

4 Stunden unter Rückfluß gekocht, mit A-Kohle heiß geklärt und das Lösungsmittel im Vakuum abdestilliert. Der schmierige Rückstand wird in wenig Wasser gelöst, die Lösung bei 60°C mit Kochsalz gesättigt, angesäuert und auf ca. 10°C abgekühlt. Hierbei fällt das Reaktionsprodukt aus. Zur Überführung in das Na-Salz wird der Filterrückstand mit Hilfe von Natronlauge in wenig Wasser gelöst und mit Kochsalz ausgesalzen. Nach Absaugen und Trocknen erhält man 17,5 g der Verbindung der Formel (106)

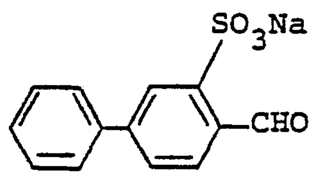

in Form eines weißen Pulvers.

## Beispiel 2

Man verfährt im wesentlichen gemäß Beispiel 1 unter Verwendung der entsprechenden 2-(4'-Diethoxyphosphonomethylphenylbenzoxazole der allgemeinen Formel (107)

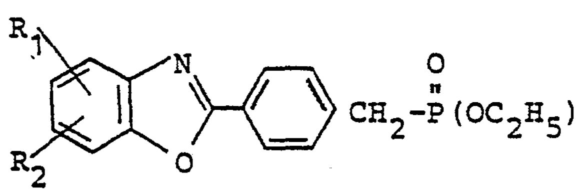

und der entsprechenden Aldehyde der allgemeinen Formel (108)

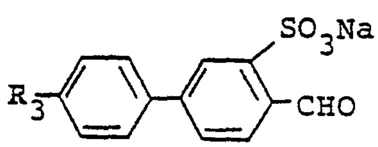

und erhält die in Tabelle I aufgeführten Verbindungen der allgemeinen Formel (109) hauptsächlich in Form ihrer Na-Salze.

(109) 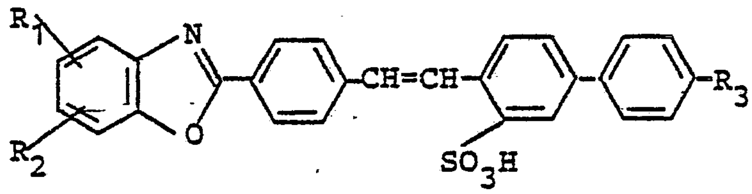

Tabelle 1

| Formel Nr. | $R_1$ | $R_2$ | $R_3$ | Pulveraspekt | Fluoreszensfarbe einer Lösung in DMF |
|---|---|---|---|---|---|
| (110) | 5-Cl | H | H | grün-gelb | neutral blau |
| (111) | 7-Cl | H | H | grün-gelb | neutral blau |
| (112) | 5-$CH_3$ | 6-$CH_3$ | H | gelb | neutral blau |
| (113) | 5-COOH | H | H | gelb | neutral blau |
| (114) | 5-tert. Butyl | H | H | gelb | neutral blau |
| (115) | H | H | Cl | grün-gelb | neutral blau |
| (116) | H | H | $SO_3H$ | gelb | neutral blau |
| (117) | H | H | COOH | gelb | neutral blau |
| (118) | 5-$CH_3$ | H | Cl | gelb | neutral blau |
| (119) | 5-$CH_3$ | H | $SO_3H$ | gelb | neutral blau |
| (120) | 5-$CH_3$ | H | COOH | gelb | neutral blau |
| (121) | 5-$CH_3$ | 7-$CH_3$ | Cl | gelb | neutral blau |
| (122) | 5-$CH_3$ | 6-$CH_3$ | Cl | gelb | neutral blau |
| (123) | 5-COOH | H | Cl | grün-gelb | neutral blau |
| (124) | 5-COOH | H | COOH | gelb | neutral blau |
| (125) | 5-$CH_3$ | 7-$CH_3$ | COOH | gelb | neutral blau |
| (126) | 5-$CH_3$ | 7-$CH_3$ | $SO_3H$ | gelb | neutral blau |
| (127) | 5-tert. Butyl | H | COOH | gelb | neutral blau |

Beispiel 3

Herstellung der Verbindung der Formel (101) nach der Anil-Methode.

Zu einer Lösung von 4,4 g 2-(p-Tolyl)-benzoxazol und 10 g der Verbindung der Formel

(128) 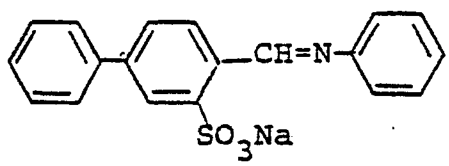

in 100 ml wasserfreiem Dimethylformamid trägt man bei 50°C unter heftigem Rühren und Überleiten von Stickstoff 14,2 g Kalium-t-butylat ein, wobei Violettfärbung eintritt. Man rührt dann 3 Stunden bei 70 bis 75°C nach, kühlt auf Raumtemperatur ab und verdünnt mit 500 ml Wasser. Das ausgefallene Produkt der Formel (101) als Kalium-Salz wird abgenutscht und mit heißem Wasser gewaschen. Zur Reinigung wird dieses mit Isopropanol ausgekocht, abgesaugt und bei 150°C getrocknet. Man erhält 6,9 g der Verbindung der Formel (101) als Kaliumsalz.

Das Natriumsalz des 4-Phenyl-benzaldehyd-2-sulfonsäure-anils (128) erhält man wie folgt:

43 g rohes Natriumsalz des 4-Phenyl-2-sulfobenzaldehyds werden in 400 ml Ethylenglykolmonomethylether kurz aufgekocht und die Lösung klärfiltriert zur Entfernung unlöslischer Salze. Man versetzt das Filtrat mit 18 g frisch dest. Anilin, erhitzt 7 Stunden bei Rückflußtemperatur und klärt erneut. Das Lösungsmittel wird im Vakuum abdestilliert und der Rückstand mit wenig Aceton gewaschen. Nach Umkristallisieren aus Wasser unter Zuhilfenahme von Kochsalz und Trocknen bei 100°C werden 36 g der Verbindung der Formel (128) erhalten.

In analoger Weise erhält man aus 4-p-Chlorphenyl-benzaldehyd-2-sulfonsäure, Na-Salz das Anil der Formel

(129) 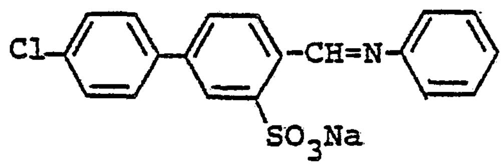

und aus 4-Formyl-biphenyl-3,4'-disulfonsäure, di-Na-Salz das Anil der Formel

(130) 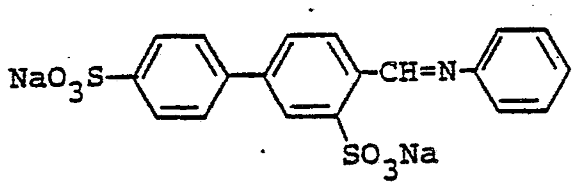

Beispiel 4

Eine Mischung von 3 g der Verbindung der Formel (101) und 60 ml konzentrierter Schwefelsäure wird bei 50°C eine Stunde gerührt. Die erhaltene klare Lösung wird auf 150 g Eis ausgetragen. Man saugt ab, löst den Filterrückstand

mit Hilfe von wenig 10 %iger Natronlauge in Wasser und salzt das entstandene di-Na-Salz mit Hilfe von Kochsalz aus. Nach dem Trocknen bei 100°C werden 2,7 g der Verbindung der Formel

(131) 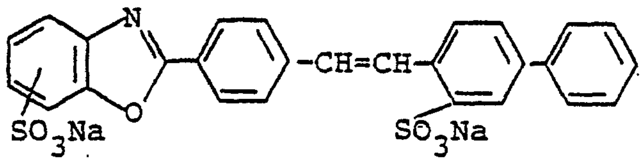

als gelbes Pulver erhalten.

Beispiel 5

Gebleichter Baumwollstoff wird im Flottenverhältnis 1:20 bei 30-60°C in einer alkalisch reagierenden Waschflotte gewaschen, die im Liter 5-10 g eines handelsüblichen anionaktiven Waschmittels enthält, in das 0,01 g eines Aufhellers der Formeln (101), (104), (105), (112), (117), (120) oder (131) eingearbeitet worden waren, sowie Natriumhypochloritlösung in einer Menge, die 0,05 bis 0,2 g aktivem Chlor pro Liter entspricht. Man erzielt hervorragend klare Aufhellungen, die sich durch sehr gute Licht-, Peroxid- und Chloritechtheiten auszeichnen.

Beispiel 6

Ein Polyamidfasergewebe wird im Flottenverhältnis 1:20 bei 30-60°C in einer alkalisch reagierenden Waschflotte gewaschen, die im Liter 5-10 g eines handelsüblichen anionaktiven Waschmittels enthält, in das 0,01 g eines Aufhellers der Formeln (101), (104),

(105), (112), (117), (120) oder (131) eingearbeitet worden waren, sowie Natriumhypochloritlösung in einer Menge, die 0,05 bis 0,2 g aktivem Chlor pro Liter entspricht. Man erzielt hervorragend klare Aufhellungen, die sich durch sehr gute Licht-, Peroxid- und Chloritechtheiten auszeichnen.

Beispiel 7

Ungebleichtes Baumwollgarn wird eine Stunde im Flottenverhältnis 1:20 bei 85-95°C in einem wäßrigen Bad behandelt, welches im Liter 0,08 g eines Aufhellers der Formeln (101), (104), (105), (110), (112), (117), (120), (125), (126) oder (131) und 2 g Natriumchlorit enthält. Das behandelte Garn zeigt nach dem Spülen und Trocknen eine klare Aufhellung von guter Lichtechtheit.

Beispiel 8

Gewebe aus Polyamid 66-Fasern ("Perlon") werden 30 Minuten im Flottenverhältnis 1:40 bei 80-90°C in einem wäßrigen Bad behandelt, das im Liter 0,15 g eines Aufhellers der Formeln (101), (104), (105), (110), (112), (117), (120), (125) oder (126) enthält. Nach dem Spülen und Trocknen zeigen die Gewebe eine starke, klare Aufhellung mit guter Licht- und Chloritechtheit.

Patentansprüche

1. Benzoxazolylstilbene der Formel

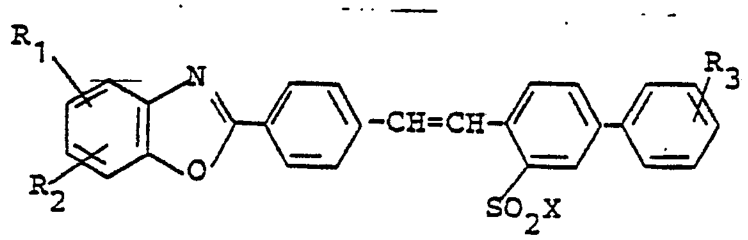

(I)

worin

$R_1$ Wasserstoff, Alkyl, Halogen, Alkoxy, Cycloalkyl, Aralkyl, Alkylsulfonyl, Phenylsulfonyl, COOV, $SO_3W$, CONVW oder $SO_2NVW$,

$R_2$ Wasserstoff, Alkyl, Alkoxy, Halogen,

$R_1$ und $R_2$ gemeinsam einen annelierten carbocyclischen Ring,

$R_3$ Wasserstoff, Halogen, COOV, $SO_3W$, CONVW oder $SO_2NVW$ und

X NVW, Z oder OV,

V Wasserstoff oder Z,

W Wasserstoff oder Alkyl und

Z Alkyl oder Phenyl bedeuten,

wobei die vorstehend genannten Kohlenwasserstoff- und Alkoxyreste sowie die gegebenenfalls anellierten Ringsysteme durch in der Aufhellerchemie übliche Substituenten substituiert sein können.

2. Benzoxazolylstilbene gemäß Anspruch 1, die in Form der freien Säure der Formel

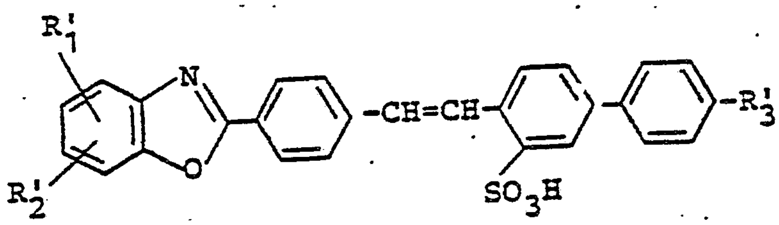

entsprechen, worin

$R_1'$ Wasserstoff, $C_1$-$C_4$-Alkyl, $C_1$-$C_4$-Alkoxy, Chlor, COOH oder $SO_3H$,

$R_2'$ Wasserstoff, $C_1$-$C_4$-Alkyl oder Chlor und

$R_3'$ Wasserstoff, Chlor, COOH oder $SO_3H$ bedeuten.

3. Benzoxozolylstilben der Formel

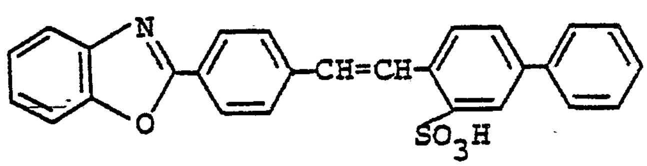

4. Verfahren zur Herstellung von Benzoxazolylstilbenen gemäß Anspruch 1, dadurch gekennzeichnet, daß man Verbindungen der Formel

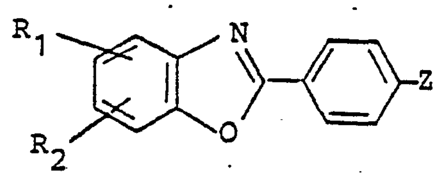

(III)

in der $R_1$ und $R_2$ die angegebene Bedeutung haben und

Z für eine Gruppierung der Formeln

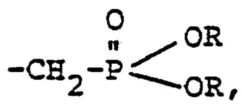 , 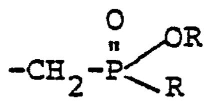 , 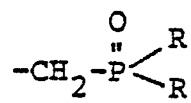 oder

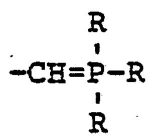

steht, in welchen R ein Alkyl-, Aralkyl, Cycloalkyl oder Arylrest ist, mit Verbindungen der Formel

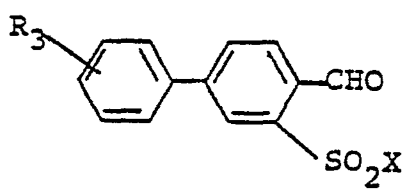

in der $R^3$ und X die angegebene Bedeutung haben, in Gegenwart einer starken Base in an sich bekannter Weise umsetzt.

5. Verfahren zur Herstellung von Benzoxazolylstilbenen gemäß Anspruch 1, dadurch gekennzeichnet, daß man Verbindungen der Formel

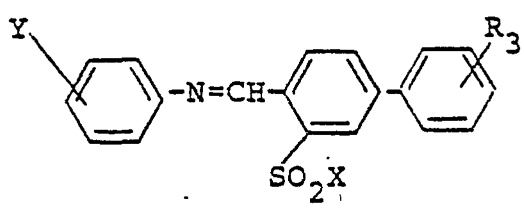

(V)

worin Y für H oder Cl steht und $R_3$ und X die angebebene Bedeutung haben,
mit einer Verbindung der Formel

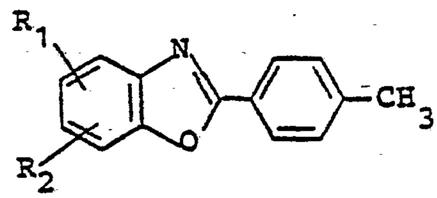

in der $R_1$ und $R_2$ die angegebene Bedeutung haben, in Gegenwart einer starken Base kondensiert.

6. Verfahren zum optischen Aufhellen von organischen Materialien, dadurch gekennzeichnet, daß man Verbindungen gemäß Anspruch 1 verwendet.

7. Optische Aufhellungsmittel enthaltend Verbindungen gemäß Anspruch 1.

Europäisches Patentamt

# EUROPÄISCHER RECHERCHENBERICHT

0033459

Nummer der Anmeldung

EP 81100328.4

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| | DE - A1 - 2 848 193 (CIBA)<br>+ Patentansprüche +<br>-- | 1,4,6 | C 07 D 263/56<br>D 06 L 3/12 |
| D,X | DE - A1 - 2 525 681 (CIBA)<br>+ Seite 2, Absätze 2,3; Seite 3, Absatz 1; Seite 17, letzter Absatz; Seite 18, Absatz 1 +<br>-- | 1,2,3,5,6,7 | |
| | CH - A5 - 565 887 (CIBA)<br>+ Spalten 1,2; Patentanspruch 1 +<br>-- | 1,4,7 | RECHERCHIERTE SACHGEBIETE (Int. Cl.3) |
| | CH - A - 558 442 (SANDOZ)<br>+ Spalte 1, Zeilen 43-66; Spalte 2, Zeilen 50-66 +<br>-- | 1,4,6,7 | C 07 D 263/00<br>D 06 L 3/00 |
| | CH - A - 555 848 (SANDOZ)<br>+ Patentansprüche +<br>-- | 1,4,7 | |
| | CH - A - 555 847 (SANDOZ)<br>+ Spalte 1, Zeilen 1-13; Spalte 3, Zeilen 12-20 +<br>---- | 1,7 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 08-04-1981 | HAMMER |